(19) Europäisches Patentamt
European Patent Office
Office européen des brevets

(11) Veröffentlichungsnummer: **0 230 224 B1**

(12) **EUROPÄISCHE PATENTSCHRIFT**

(45) Veröffentlichungstag der Patentschrift: **17.07.91**

(51) Int. Cl.$^5$: **C07C 263/04**

(21) Anmeldenummer: **87100086.5**

(22) Anmeldetag: **07.01.87**

# (54) Verfahren zur Herstellung von Alkoxycarbonylisocyanaten.

(30) Priorität: **18.01.86 DE 3601376**

(43) Veröffentlichungstag der Anmeldung:
**29.07.87 Patentblatt 87/31**

(45) Bekanntmachung des Hinweises auf die Patenterteilung:
**17.07.91 Patentblatt 91/29**

(84) Benannte Vertragsstaaten:
**BE CH DE FR GB IT LI**

(56) Entgegenhaltungen:

**HOUBEN-WEYL: "Methoden der Organischen Chemie", Band E4, "Kohlensäure-Derivate", 1983, Georg Thieme Verlag, Stuttgart, DE**

(73) Patentinhaber: **BAYER AG**

**W-5090 Leverkusen 1 Bayerwerk(DE)**

(72) Erfinder: **Krebs, Andreas, Dr.**
**Am Gartenfeld 70**
**W-5068 Odenthal(DE)**
Erfinder: **Hagemann, Hermann, Dr.**
**Kandinskystrasse 52**
**W-5090 Leverkusen(DE)**

**EP 0 230 224 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Alkoxycarbonylisocyanaten aus N-unsubstituierten Carbamidsäureestern oder Harnstoffdicarbonsäureestern.

Es ist bereits bekannt, daß Ethoxycarbonylisocyanate durch Umsetzung von Stickstofftricarbonsäuretriethylester mit Phosphorpentoxid erhalten werden können (O. Diels und B. Wolf, Ber. Dtsch. Chem. Ges. 39, 688 (1906)). Man kann Alkoxycarbonylisocyanate auch aus N-unsubstituierten Carbamidsäureestern und Oxalylchlorid, aus Alkoxycarbonylisocyaniddichloriden und Methansulfonsäure und aus Alkoholen und Chlorcarbonylisocyanat oder N-Chlorcarbonyl-iminokohlensäurechloridester erhalten (siehe H. Hagemann in Houben-Weyl, Band E4, Seite 1238 (1983)). Allen diesen Verfahren ist gemeinsam, daß sie mindestens einen Einsatzstoff benötigen, der in technischen Mengen nur schwierig oder gar nicht zugänglich ist.

Weiterhin ist bekannt, daß bei der Phosgenierung von N-unsubstituierten Carbamidsäureestern Harnstoffdicarbonsäureester entstehen (siehe A. Botta in Houben-Weyl, a.a.O., Seite 1321).

Es wurde nun ein Verfahren zur Herstellung von Alkoxycarbonylisocyanaten aus Carbamidsäureestern gefunden, das dadurch gekennzeichnet ist, daß man Carbamidsäureester der Formel

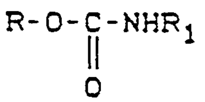

(I)

in der

R für einen gegebenenfalls substituierten Alkyl- , Cycloalkyl- oder Aralkylrest und

$R_1$ für Wasserstoff oder

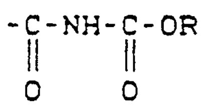

steht, wobei R die oben angegebene Bedeutung hat,

mit Phosgen umsetzt und ein N-disubstituiertes Formamid als Katalysator zugibt.

In Formel (I) kann R beispielsweise für einen Alkylrest mit 1 bis 20 C-Atomen, einen Cycloalkylrest mit 4 bis 20 C-Atomen oder einen Aralkylrest mit 7 bis 20 C-Atomen stehen. Alle diese Reste können substituiert sein, bei spielsweise durch einen oder mehrere $C_1$-$C_{10}$-Alkylreste, ein oder mehrere Halogenatome oder ein oder mehrere $C_1$-$C_{10}$-Alkoxygruppen. Von den Halogenatomen sind dabei Fluor, Chlor und Brom bevorzugt. Vorzugsweise handelt es sich beim Rest R in Formel (I) um eine geradkettige oder verzweigte Alkylgruppe, die gegebenenfalls durch Fluor, Chlor oder eine $C_1$-$C_5$-Alkoxygruppe substituiert ist und insgesamt 1 bis 10 C-Atome enthält, um eine Cycloalkylgruppe, die gegebenenfalls durch $C_1$-$C_5$-Alkyl, Fluor, Chlor oder $C_1$-$C_5$-Alkoxy substituiert ist und insgesamt 5 bis 12 C-Atome enthält oder einen Aralkylrest, der gegebenenfalls durch $C_1$-$C_5$-Alkyl, Fluor, Chlor oder $C_1$-$C_5$-Alkoxy substituiert ist und insgesamt 7 bis 12 C-Atome enthält. Beispiele für R sind Methyl, Ethyl, n-Propyl, Isopropyl, n-Butyl, Isobutyl, sec.-Butyl, n-Pentyl, 3-Methylbutyl, 2,2-Dimethylpropyl, n-Hexyl, 1,3-Dimethylbutyl, 2-Ethylbutyl, 2-Ethylhexyl, Nonyl, Decyl, Dodecyl, Tetradecyl, Hexadecyl, Octadecyl, Cyclohexyl, Cyclohexylmethyl, 4-tert.-Butylcylcohexyl, Menthyl, Benzyl, Phenylethyl, 2-Methoxyethyl, 2-Ethoxyethyl, 2-Propoxyethyl, 2-Butoxyethyl, 2-(2-Ethoxyethoxy)-ethyl, 2-Chlorethyl, 4-Chlorbutyl, 2,2,2-Trichlorethyl und 2,2,2-Trifluorethyl.

Wenn in Formel (I) $R_1$ für

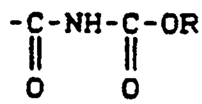

steht, handelt es sich bei den Verbindungen der Formel (I) um Harnstoffdicarbonsäureester. Diese müssen nicht notwendigerweise in beiden Teilen die gleichen Reste R enthalten, tun dies aber vorzugsweise. Von den beiden Bedeutungen von $R_1$ ist die Bedeutung $R_1$ = Wasserstoff bevorzugt.

Phosgen kann in das erfindungsgemäße Verfahren beispielsweise gasförmig oder unter Druck verflüssigt und in üblichen Qualitäten eingesetzt werden. Ausgehend von Carbamidsäureestern der Formel (I) mit $R_1$ = Wasserstoff wird für die erfindungsgemäße Umsetzung vorzugsweise pro Mol Carbamidsäureester mindestens 1 Mol Phosgen eingesetzt, ausgehend von Carbamidsäureestern der Formel (I) mit $R_1$ =

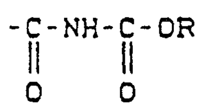

wird vorzugsweise pro Mol Carbamidsäureester ebenfalls mindestens 1 Mol Phosgen eingesetzt. Das Phosgen kann in das erfindungsgemäße Verfahren in beliebigen Überschüssen eingesetzt werden. Aus ökonomischen Gründen setzt man im allgemeinen nicht mehr als 10 Mole Phosgen pro Mol Carbamidsäureester ein, oder man führt unumgesetztes Phosgen zurück, beispielsweise indem man den Abgasstrom durch einen unter den Kondensationspunkt des Phosgens gekühlten Rückflußkühler leitet.

Für das erfindungsgemäße Verfahren kommen als Katalysatoren N-disubstituierte Formamide in Frage. Diese können am Stickstoffatom zwei gleiche oder verschiedene Substituenten enthalten, bei denen es sich beispielsweise um geradkettige oder verzweigte Alkylgruppen mit insgesamt 1 - 20 C-Atomen handeln kann, aber auch um Cycloalkylgruppen mit 4 bis 12 C-Atomen, um Aralkylgruppen mit 7 bis 12 C-Atomen und/oder um Arylgruppen mit 6 bis 12 C-Atomen. Alle diese Gruppen können gegebenenfalls ihrerseits substituiert sein. Die Alkylgruppen können gegebenenfalls durch Fluor, Chlor und/oder $C_6$-$C_{10}$-Aryl substituiert sein, die Cycloalkylgruppen gegebenenfalls durch Fluor, Chlor, $C_1$-$C_{10}$-Alkyl und/oder $C_6$-$C_{10}$-Aryl, die Aralkylgruppen gegebenenfalls durch Fluor, Chlor, $C_1$-$C_{10}$-Alkyl (im Arylteil) oder $C_6$-$C_{10}$-Aryl (im Alkylteil) und die Arylgruppen gegebenenfalls durch Fluor, Chlor, $C_1$-$C_{10}$-Alkyl oder $C_4$-$C_8$-Cycloalkyl.

Für das erfindungsgemäße Verfahren kommen als Katalysatoren beispielsweise auch solche N-disubstituierten Formamide in Frage, bei denen das Stickstoffatom Bestandteil eines heterocyclischen Ringsystems ist, beispielsweise eines Pyrrolidin-, Piperidin- oder Morpholinringsystems.

Als Beispiele für Katalysatoren seien genannt: N,N-Dimethylformamid, N,N-Diethylformamid, N,N-Di-(n)-propylformamid, N,N-Isopropylformamid, N,N-Di-(n)-butylformamid, N,N-Di-sec.-butylformamid, N,N-Diisobutylformamid, N,N-Di-(n)-hexylformamid, N,N-Di-(2-ethylhexyl)-formamid, N,N-Didodecylformamid, N-Butyl-N-ethylformamid, N-Methyl-N-stearylformamid, N-Cyclohexyl-N-methylformamid, N,N-Di-cyclohexylformamid, N-Methylformanilid, N-Butylformanilid, N,N-Dibenzylformamid, N-Formylpyrrolidin, N-Formylpiperidin und N-Formylmorpholin.

Als Katalysator können auch bifunktionelle Formamide eingesetzt werden, beispielsweise N,N'-Dimethyl-N,N'-diformyl-ethylendiamin.

Die Menge des Katalysators ist im allgemeinen nicht kritisch. Geeignete Anwendungsmengen sind beispielsweise 0,02 bis 50 Mol-% Katalysator, bezogen auf die eingesetzte Verbindung der Formel (I). Vorzugsweise beträgt diese Menge 0,2 bis 5 Mol-%.

Die Zugabe des Katalysators kann zu verschiedenen Zeitpunkten erfolgen. Man kann den Katalysator beispielsweise von vornherein zusetzen, d.h. bevor das Phosgen zugesetzt wird. Häufig ist es ohne irgendwelche Nachteile in Kauf nehmen zu müssen auch möglich den Katalysator erst im Laufe der Reaktion zuzusetzen, insbesondere dann, wenn von Verbindungen der Formel (I) ausgegangen wird, in denen $R_1$ für Wasserstoff steht.

Das erfindungsgemäße Verfahren kann in Anwesenheit oder Abwesenheit von Lösungsmitteln durchgeführt werden. Häufig ist die Anwesenheit von Lösungsmitteln vorteilhaft, insbesondere um einen guten Wärmeübergang und/oder einen gut rührbaren Ansatz zu haben. Als Lösungsmittel kommen solche in Betracht, die unter den Reaktionsbedingungen nahezu oder vollständig inert sind. Geeignet sind beispielsweise aliphatische Kohlenwasserstoffe, vorzugsweise solche mit Siedepunkten zwischen 80 und 300 °C, wie Heptan, Octan, Methylcylohexan, Ligroin und Waschbenzin, aromatische Kohlenwasserstoffe wie Toluol, Xylol, Isopropylbenzol, 1-Methylnaphthalin und Biphenyl, halogenierte aliphatische Kohlenwasserstoffe wie Tetrachlormethan, 1,2-Dichlorethan, 1,1,1-Trichlorethan und 1,1,2,3-Tetrachlorethan, halogenierte aromatische Kohlenwasserstoffe wie Chlorbenzol, 1,2-Dichlorbenzol, 1,2,4-Trichlorbenzol und 1-Chlornaphthalin sowie Ester wie Essigsäureethylester, Essigsäurebutylester, Essigsäure-2-ethylhexylester, 1,2-Diacetoxyethan, Benzoesäureethylester und Kohlensäurediethylester. Besonders bevorzugte Lösungsmittel sind Chlorbenzol und 1,2-Dichlorbenzol. Sofern Lösungsmittel eingesetzt werden, können diese beispielsweise in Mengen von 10 bis 1000 Gew.-%, bezogen auf den Carbamidsäureester der Formel (I) eingesetzt werden.

Das erfindungsgemäße Verfahren kann beispielsweise bei Temperaturen von 20 bis 200 °C durchgeführt werden.

Vorzugsweise beträgt die Reaktionstemperatur 60 bis 180 °C. Es kann bei Normaldruck, erhöhtem und vermindertem Druck gearbeitet werden. Vorzugsweise wird bei Normaldruck oder schwach erhöhtem Druck, beispielsweise bei 1 bis 5 bar gearbeitet. Der Carbamidsäureester der Formel (I) kann nicht nur in Form einer Lösung in einem der zuvor angegebenen Lösungsmittel, sondern auch in geschmolzener Form erfindungsgemäß mit Phosgen umgesetzt werden, wobei der Katalysator und gegebenenfalls ein Lösungsmittel auch erst zu einem späteren Zeitpunkt, d.h. nach der ersten Zugabe von Phosgen, zugesetzt werden kann.

Die Aufarbeitung des nach der erfindungsgemäßen Umsetzung vorliegenden Reaktionsgemisches kann beispielsweise durch fraktionierte Destillation bei normalem oder vermindertem Druck erfolgen. Das bei der Aufarbeitung gegebenenfalls zurückgewonnene Lösungsmittel kann für weitere Ansätze wieder verwendet werden. Nach der Abtrennung leicht flüchtiger Bestandteile des Reaktionsgemisches und des hergestellten Alkoxycarbonylisocyanates verbleibt im allgemeinen ein Rückstand, der den eingesetzten Katalysator und Verbindungen enthalten kann, die bei den erfindungsgemäß anzuwendenden Reaktionsbedingungen in

Alkoxycarbonylisocyanate überführt werden können. Es ist deshalb im allgemeinen vorteilhaft, einen derartigen Rückstand ganz oder teilweise, gegebenenfalls unter Zugabe frischen Katalysators, einem weiteren Ansatz zur Durchführung des erfindungsgemäßen Verfahrens zuzuführen.

Das erfindungsgemäße Verfahren bietet gegenüber der bekannten Umsetzung von Carbamidsäureester mit Oxalylchlorid den Vorteil, daß als einziges gasförmiges Reaktionsprodukt Chlorwasserstoff entsteht, während bei der Reaktion mit Oxalylchlorid ein Gemisch aus Kohlenmonoxid und Chlorwasserstoff anfällt. Außerdem werden für das erfindungsgemäße Verfahren Einsatzstoffe benötigt, die auch in technischen Mengen gut zugänglich sind.

Es ist außerordentlich überraschend, daß nach dem erfindungsgemäßen Verfahren Alkoxycarbonylisocyanate so gut hergestellt werden können, denn gemäß A. Botta in Houben-Weyl, a.a.O., konnte lediglich die Bildung von Harnstoffdicarbonsäureestern erwartet werden.

Die nach dem erfindungsgemäßen Verfahren erhältlichen Verbindungen sind bekannte Alkoxycarbonylisocyanate, die beispielsweise als wertvolle Zwischenprodukte in der organischen Chemie verwendet werden können, oder auch als Stabilisatoren für freie Isocyanatgruppen enthaltende Polyurethane (siehe DE-OS 25 39 728).

## Beispiele

### Beispiel 1

86,5 g Carbamidsäure-2-ethylhexylester wurden geschmolzen vorgelegt und bei 80 °C innerhalb von 6 Stunden 81 g Phosgen in die Schmelze eingeleitet. Dann wurden 250 g Chlorbenzol und 3,1 g (= 2 Mol-%) Methylstearylformamid zugegeben, unter Einleiten von weiteren 108 g Phosgen auf 130 °C erhitzt und 6 Stunden unter Rückflußkühlung gerührt. Überschüssiges Phosgen wurde danach bei 130 °C mit Stickstoff ausgeblasen. Anschließend wurde durch eine 40 cm lange Vigreux-Kolonne destilliert, wobei 74,7 g (= 75 % der Theorie) 2-Ethylhexyloxycarbonylisocyanat mit einem Siedepunkt von 62 bis 66 °C bei 0,8 mbar erhalten wurden.

### Beispiel 2

Es wurde wie in Beispiel 1 verfahren, jedoch wurden als Katalysator 3,1 g (= 4 Mol-%) Di-n-butylformamid verwendet. Es wurden 70,7 g (= 71 % der Theorie) 2-Ethylhexyloxycarbonylisocyanat mit einem Siedepunkt von 42 bis 46 °C bei 0,07 mbar erhalten.

### Beispiel 3

Es wurde wie in Beispiel 1 verfahren, jedoch wurden anstelle von Chlorbenzol 250 g Butylacetat eingesetzt. Es wurden 59,4 g (= 60 % der Theorie) 2-Ethylhexyloxycarbonylisocyanat mit einem Siedepunkt von 68 bis 70 °C bei 0,9 mbar erhalten.

### Beispiel 4

58,5 g Carbamidsäure-n-butylester wurden als Schmelze vorgelegt und bei 80 °C innerhalb von 6 Stunden 86 g Phosgen in diese Schmelze eingeleitet. Dann wurden 3,1 g (= 2 Mol-%) Methylstearylformamid und 125 g Chlorbenzol zugegeben. Dann wurden weitere 113 g Phosgen innerhalb von 6 Stunden bei 130 °C eingeleitet und anschließend überschüssiges Phosgen mit Stickstoff ausgeblasen. Die Destillation des Reaktionsgemisches ergab 32,2 g (= 45 % der Theorie) Butyloxycarbonylisocyanat mit einem Siedepunkt von 52 bis 54 °C bei 24 mbar.

### Beispiel 5

45,5 g Carbamidsäure-δ-chlorbutylester wurden als Schmelze vorgelegt und bei 80 °C innerhalb von 6 Stunden 63 g Phosgen in die Schmelze eingeleitet. Sodann wurden 150 g Chlorbenzol und 0,36 g (= 0,4 Mol-%) Methylstearylformamid zugegeben und weitere 16 Stunden bei 130 °C weitere 159 g Phosgen eingeleitet. Überschüssiges Phosgen wurde anschließend mit Stickstoff ausgetrieben. Bei der anschließenden Destillation wurden 23,7 g δ-Chlorbutyloxycarbonylisocyanat mit einem Siedepunkt von 86 bis 109 °C bei 20 mbar erhalten, was einer Ausbeute von 44 % der Theorie entsprach.

### Beispiel 6

35,7 g Carbamidsäure-2-methoxyethylester wurden analog Beispiel 5 zu 2-Methoxyethyloxycarbonylisocyanat phosgeniert. Der Siedepunkt des Produktes betrug 71 bis 74 °C bei 20 mbar, die Ausbeute 12,9 g (= 30 % der Theorie).

### Beispiel 7

18,0 g Harnstoffdicarbonsäurediethylester wurden in 50 g 1,2-Dichlorbenzol vorgelegt, 1,3 g Methylstearylformamid zugegeben und 3 Stunden bei 130 °C und 2 Stunden bei 180 °C insgesamt 52 g Phosgen eingeleitet, wobei gleichzeitig das Produkt abdestilliert wurde. Bei der erneuten Destillation des Rohproduktes wurden 6,9 g (30 % der Theorie) Ethoxycarbonylisocyanat mit einem Siedepunkt von 25 bis 35 °C bei 14 mbar erhalten.

### Beispiel 8

138,6 g Carbamidsäure-2-ethylhexylester wurden geschmolzen vorgelegt und bei 80 ° C innerhalb von 3,25 Stunden 93 g Phosgen eingeleitet, wobei der Abgasstrom durch einen auf -78 ° C gekühlten Rückflußkühler geleitet wurde. Dann wurden 800 g Chlorbenzol sowie 5,0 g Methylstearylformamid zugegeben, auf 120 ° C erhitzt und innerhalb von 3 Stunden 48 g Phosgen eingeleitet. Es wurde noch 1 Stunde bei 130 ° C nachgerührt und anschließend überschüssiges Phosgen mit Stickstoff ausgeblasen. Die anschließende Destillation ergab 135,5 g (= 85 % der Theorie) 2-Ethylhexyloxycarbonylisocyanat mit einem Siedepunkt von 45 - 60 ° C bei 0,13 - 0,4 mbar.

## Patentansprüche

1. Verfahren zur Herstellung von Alkoxycarbonylisocyanaten aus Carbamidsäureestern, dadurch gekennzeichnet, daß man Carbamidsäureester der Formel

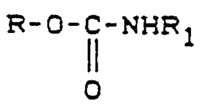

(I)

in der

R für einen gegebenenfalls substituierten Alkyl-, Cycloalkyl- oder Aralkylrest und

$R_1$ für Wasserstoff oder

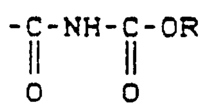

steht, wobei R die oben angegebene Bedeutung hat,

mit Phosgen umsetzt und ein N-disubstituiertes Formamid als Katalysator zugibt.

2. Verfahren nach Anspruch 1, dadurch gekennzeichnet, daß in Formel (I) R für einen gegebenenfalls substituierten Alkylrest mit 1 bis 20 C-Atomen, einen Cycloalkylrest mit 4 bis 20 C-Atomen oder einen Aralkylrest mit 7 bis 20 C-Atomen steht.

3. Verfahren nach Ansprüchen 1 und 2, dadurch gekennzeichnet, daß in Formel (I) im Rest R als Substituenten ein oder mehrere $C_1$- bis $C_{10}$-Alkylreste, ein oder mehrere Halogenatome und/oder ein oder mehrere $C_1$- bis $C_{10}$-Alkoxygruppen vorliegen.

4. Verfahren nach Ansprüchen 1 bis 3, dadurch gekennzeichnet, daß in Formel (I) $R_1$ für Wasserstoff steht.

5. Verfahren nach Ansprüchen 1 bis 4, dadurch gekennzeichnet, daß sowohl im Falle $R_1$ = Wasserstoff als auch im Falle

$R_1$ = 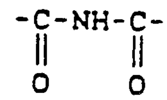

mindestens 1 Mol Phosgen jeweils pro Mol Carbamidsäureester eingesetzt werden.

6. Verfahren nach Ansprüchen 1 bis 5, dadurch gekennzeichnet, daß das N-disubstituierte Formamid als Substituenten geradkettige oder verzweigte Alkylgruppen mit 1 bis 20 C-Atomen, Cycloalkylgruppen mit 4 bis 12 C-Atomen, Aralkylgruppen mit 7 bis 12 C-Atomen und/oder Arylgruppen mit 6 bis 12 C-Atomen enthält, die gegebenenfalls ihrerseits substituiert sind.

7. Verfahren nach Anspruch 6, dadurch gekennzeichnet, daß es sich bei dem N-disubstituierten Formamid um N,N-Dimethylformamid, N,N-Diethylformamid, N,N-Di-(n)-propylformamid, N,N-Isopropylformamid, N,N-Di-(n)-butylformamid, N,N-Di-sec.-butylformamid, N,N-Diisobutylformamid, N,N-Di-(n)-hexylformamid, N,N-Di-(2-ethylhexyl)-formamid, N,N-Didodecylformamid, N-Butyl-N-ethylformamid, N-Methyl-N-stearylformamid, N-Cyclohexyl-N-methylformamid, N,N-Dicyclohexylformamid, N-Methylformanilid, N-Butylformanilid, N,N-Dibenzylformamid, N-Formylpyrrolidin, N-Formylpiperidin, N-Formylmorpholin oder N,N'-Dimethyl-N,N'-diformylethylendiamin handelt.

8. Verfahren nach Ansprüchen 1 bis 7, dadurch gekennzeichnet, daß man den Katalysator in einer Menge von 0,02 bis 50 Mol-%, bezogen auf die eingesetzte Verbindung der Formel (I), einsetzt.

9. Verfahren nach Ansprüchen 1 bis 8, dadurch gekennzeichnet, daß man es bei Temperaturen von 20 bis 200 ° C durchführt.

## Claims

1. A process for the production of alkoxycarbonyl isocyanates from carbamic acid esters, characterized in that carbamic acid esters corresponding to the following formula

$$R-O-\underset{\underset{O}{\|}}{C}-NHR_1 \qquad (I)$$

in which

R is an optionally substituted alkyl, cycloalkyl or aralkyl radical and

$R_1$ is hydrogen or

$$-\underset{\underset{O}{\|}}{C}-NH-\underset{\underset{O}{\|}}{C}-OR,$$

where R is as defined above,

are reacted with phosgene and an N-disubstituted formamide is added as catalyst.

**2.** A process as claimed in claim 1, characterized in that, in formula (I), R is an optionally substituted $C_{1-20}$ alkyl radical , a $C_{4-20}$ cycloalkyl radical or a $C_{7-20}$ aralkyl radical.

**3.** A process as claimed in claims 1 and 2, characterized in that, in formula (I), one or more $C_{1-10}$ alkyl radicals, one or more halogen atoms and/or more or more $C_{1-10}$ alkoxy groups are present as substituents in the radical R.

**4.** A process as claimed in claims 1 to 3, characterized in that, in formula (I), $R_1$ is hydrogen.

**5.** A process as claimed in claims 1 to 4, characterized in that, both where $R_1$ = hydrogen and where

$$R_1 = -\underset{\underset{O}{\|}}{C}-NH-\underset{\underset{O}{\|}}{C}OR,$$

at least 1 mol phosgene is used per mol carbamic acid ester.

**6.** A process as claimed in claims 1 to 5, characterized in that the N-disubstituted formamide contains as substituents linear or branched $C_{1-20}$ alkyl groups, $C_{4-20}$ cycloalkyl groups, $C_{7-12}$ aralkyl groups and/or $C_{6-12}$ aryl groups which in turn may optionally be substituted.

**7.** A process as claimed in claim 6, characterized in that the N-disubstituted formamide is N,N-dimethyl formamide, N,N-diethyl formamide, N,N-di-(n)-propyl formamide, N,N-isopropyl formamide, N,N-di-(n)-butyl formamide, N,N-di-sec.-butyl formamide, N,N-diisobutyl formamide, N,N-di-(n)-hexyl formamide, N,N-di-(2-ethylhexyl)-formamide, N,N-didodecyl formamide, N-butyl-N-ethyl formamide, N-methyl-N-stearyl formamide, N-cyclohexyl-N-methyl formamide, N,N-dicyclohexyl formamide, N-methyl formanilide, N-butyl formanilide, N,N-dibenzyl formamide, N-formyl pyrrolidine, N-formyl piperidine, N-formyl morpholine or N,N'-dimethyl-N,N'-diformyl ethylenediamine.

**8.** A process as claimed in claims 1 to 7, characterized in that the catalyst is used in a quantity of 0.02 to 50 mol-%, based on the compound of formula (I) used.

**9.** A process as claimed in claims 1 to 8, characterized in that it is carried out at temperatures of 20 to 200 °C.

**Revendications**

**1.** Procédé de préparation d'isocyanates d'alkoxycarbonyle d'esters d'acide carbamique, caractérisé en ce qu'on fait réagir avec le phosgène des esters d'acide carbamique de formule

$$R-O-\underset{\underset{O}{\|}}{C}-NHR_1 \qquad (I)$$

dans laquelle

R représente un reste alkyle, cycloalkyle ou aralkyle éventuellement substitué et

$R_1$ est l'hydrogène ou un reste

$$-\underset{\underset{O}{\|}}{C}-NH-\underset{\underset{O}{\|}}{C}-OR$$

où R a la définition indiquée ci-dessus,

et on ajoute comme catalyseur un formamide disubstitué sur l'azote.

**2.** Procédé suivant la revendication 1, caractérisé en ce que dans la formule (I), R représente un reste alkyle de 1 à 20 atomes de carbone éventuellement substitué, un reste cycloalkyle de 4 à 20 atomes de carbone ou un reste aralkyle de 7 à 20 atomes de carbone.

**3.** Procédé suivant les revendications 1 et 2, caractérisé en ce que dans la formule (I), il y a comme substituants dans le reste R un ou plusieurs restes alkyle en $C_1$ à $C_{10}$, un ou plusieurs atomes d'halogènes et/ou un ou plusieurs groupes alkoxy en $C_1$ à $C_{10}$.

**4.** Procédé suivant les revendications 1 à 3, caractérisé en ce que dans la formule (I), $R_1$ est l'hydrogène.

**5.** Procédé suivant les revendications 1 à 4, caractérisé en ce qu'on utilise au moins 1 mole de phosgène par mole d'ester d'acide carbamique tant lorsque $R_1$ est l'hydrogène que lorsque $R_1$ représente un groupe

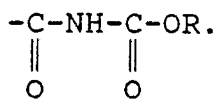

**6.** Procédé suivant les revendications 1 à 5, caractérisé en ce que le formamide disubstitué sur l'azote contient comme substituants des groupes alkyle à chaîne droite ou ramifiée ayant 1 à 20 atomes de carbone, des groupes cycloalkyle ayant 4 à 12 atomes de carbone, des groupes aralkyle ayant 7 à 12 atomes de carbone et/ou des groupes aryle ayant 6 à 12 atomes de carbone, qui sont euxmêmes substitués le cas échéant.

**7.** Procédé suivant la revendication 6, caractérisé en ce que le formamide disubstitué sur l'atome d'azote est le N,N-diméthylformamide, le N,N-diéthylformamide, le N,N-di-(n)-propylformamide, le N,N-isopropylformamide, le N,N-di-(n)-butylformamide, le N,N-di-sec.-butylformamide, le N,N-diisobutylformamide, le N,N-di-(n)-hexylformamide, le N,N-di-(2-éthylhexyl)-formamide, le N,N-di-dodécylformamide, le N-butyl-N-éthylformamide, le N-méthyl-N-stéarylformamide, le N-cyclohexyl-N-méthylformamide, le N,N-dicyclohexylformamide, le N-méthylformanilide, le N-butylformanilide, le N,N-dibenzylformamide, la N-formylpyrrolidine, la N-formylpipéridine, la N-formylmorpholine ou la N,N'-diméthyl-N,N'-diformyléthylènediamine.

**8.** Procédé suivant les revendications 1 à 7, caractérisé en ce qu'on ajoute le catalyseur en une quantité de 0,02 à 50 moles % par rapport au composé utilisé de formule (I).

**9.** Procédé suivant les revendications 1 à 8, caractérisé en ce qu'il est mis en oeuvre à des températures de 20 à 200 °C.